(19) Europäisches Patentamt
European Patent Office
Office européen des brevets

(11) Publication number : 0 445 948 A2

(12) EUROPEAN PATENT APPLICATION

(21) Application number : 91301546.7

(51) Int. Cl.$^5$ : A61K 31/557

(22) Date of filing : 26.02.91

(30) Priority : 28.02.90 GB 9004482

(43) Date of publication of application : 11.09.91 Bulletin 91/37

(84) Designated Contracting States : AT BE CH DE DK ES FR GB GR IT LI LU NL SE

(71) Applicant : G.D. Searle & Co.
P.O. Box 5110
Chicago Illinois 60680 (US)

(72) Inventor : Shield Michael
Oxford road,Botts Furlong,Stone,Aylesbury
Buckinghamshire HP 17 8PR (GB)
Inventor : Dingle John
Walnut Tree Cottage, 38 Church street
Great Shelford,Cambridge, CB2 5EL (GB)

(74) Representative : Goldin, Douglas Michael et al
J.A. KEMP & CO. 14, South Square Gray's Inn
London WC1R 5EU (GB)

(54) **Prostaglandins for inhibiting cartilage damage.**

(57) Misoprostol is used in the preparation of a medicament to inhibit cartilage damage, to reverse inhibition of cartilage synthesis, to inhibit the damaging effect of IL1 on cartilage integrity by direct damage or to inhibit cartilage synthesis.

EP 0 445 948 A2

$^{35}SO_4$ Uptake Into GAG cpm/mg x $10^3$

FIG.1

DOSE CURVE OF hr IL 1 α ON NORMAL HUMAN CARTILAGE

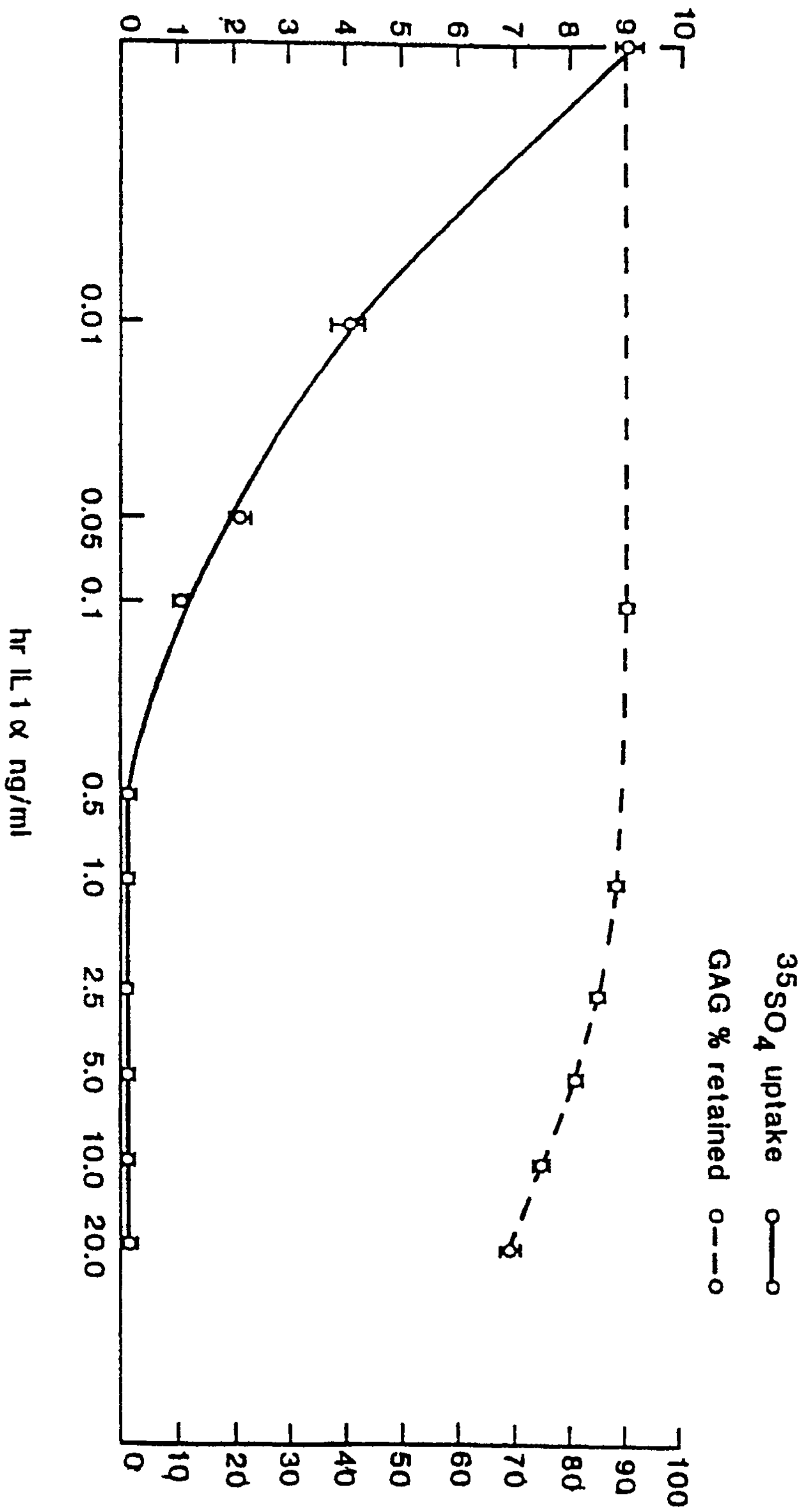


GAG Percentage Retained in Cartilage

# PROSTAGLANDINS FOR INHIBITING CARTILAGE DAMAGE

## Background Of The Invention

The invention herein is directed to the use of certain prostaglandins for inhibiting cartilage damage and cartilage synthesis inhibition in a patient susceptible to such cartilage damage and such cartilage synthesis inhibition. It has been found that the administration of a prostaglandin of the formula

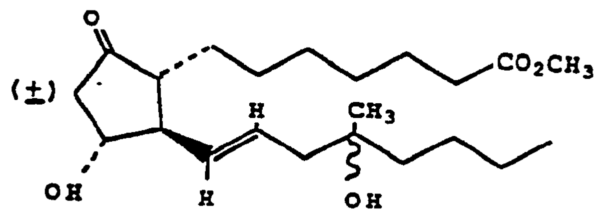

can exhibit an inhibiting affect on cartilage damage and can promote repair of human cartilage which has been rendered susceptible to cartilage damage.

The prostaglandin compounds, and their preparation are described in U.S. Patent 3,965,143 and 4,060,691. The prostaglandin compounds herein are commercially available under the USAN (United States Adopted Name) name misoprostol as a pharmaceutical which has been accepted for use in the treatment of NSAID induced ulcers in many countries, including the UnitedStates and which is commercially available by prescription in such countries.

It has been recognized for over 2 decades that cartilage is capable of degradation and synthesis of its extracellular matrix (Dingle, J. T. "The Role of Lysosomes in Connective Tissues Disease" in Hill, A. G., ed.) Modern Trends in Rheumatology, pp. 110-120, Butterworths 1966. It is now recognized that repair of cartilage damage is possible. Nakata, et al. "The Injury and Repair of Human Articular Cartilage: A Morphological Study of 192 Cases of Osteoarthritis" J. Japan. Orthop. Ass. 1986, 60, 763-775. In view of the recognition that cartilage damage as well as the repair of cartilage damage may be controllable, it would be desirable to find compounds which could be administered as pharmaceutical agents (medicaments) for the treatment of patients susceptible to or exhibiting cartilage damage or inhibition of cartilage synthesis to inhibit such cartilage damage and/or enhance the repair of cartilage damage by overcoming the inhibition of cartilage synthesis.

Non Steroidal Anti-Inflammatory Drugs (NSAIDs) have long been used for the treatment of osteoarthritis (OA) and rheumatoid arthritis (RA) to provide relief from the pain associated with such diseases and to increase the range of movement in the patients with such diseases. It has been found that some NSAIDS can inhibit cartilage synthesis or promote cartilage damage. Thus, not only can cartilage damage arise due to the malady, rheumatoid arthritis or osteoarthritis, but also the pharmaceuticals indicated for treatment of such OA and RA can also exhibit certain cartilage damaging properties.

It has been known for many years that articular cartilage chondrocytes retain their ability to synthesize matrix components throughout life and exhibit the ability to take up sulphate and form new glycosaminoglycans (GAGs). It would be desirable to identify a compound which can be used as a pharmaceutical product to enhance and/or promote the synthesis and repair of cartilage in a patient wherein cartilage has been damaged or is susceptible to damage.

It has been found that the catabolic action of the cytokine IL1(hrIL1α) plays an important role in inflammatory diseases and a role in cartilage damage. Suppression of IL1 production is possible using exogenous PGEs Bodger, et al. "Immuno Modulatory approaches To The Treatment of Inflammation" in (Johns, W. F. ed.) Section 4; Endocrinology, Immunology and Metabolic Disorders Annual Reports in Medicinal Chemistry, 1988, pp. 171-180 Academic Press Inc.; Kunkel, et al. "Arachadonic Acid Metabolites Regulate Interleukin 1 Production" Biochem. Biophys. Res. Commun. 1985, 128, 892-897.; and Numo, et al. "Present Status and New Prospectives in Non Steroidal Anti-Inflammatory Drug Therapy" Scand. J. Rheumatol. 1987, Supplement 66, 75-83. In view of the role of IL1 in cartilage degeneration it is highly desirable to find a compound which could diminish the negative effects of IL1 on cartilage degeneration.

## Summary of the Invention

The invention herein is directed to the use of a prostaglandin of the following structure to inhibit cartilage

damage

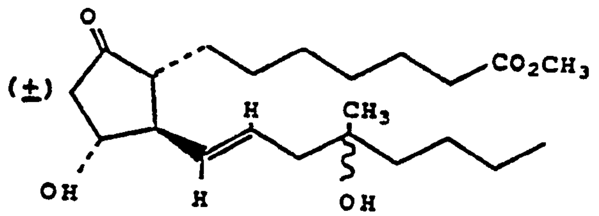

in patients susceptible to such damage. The prostaglandin is administered to the patient in an effective cartilage damaging inhibiting amount which amount is included herein to mean an amount sufficient for overcoming inhibition of cartilage synthesis.

The invention herein is also directed to a method for inhibiting the damaging affect of IL1 on cartilage integrity by administering a prostaglandin of the above structure in an effective IL1 inhibiting amount. Administering the prostaglandin inhibits the IL1 induced inhibition of cartilage synthesis and cartilage damage and promotes regeneration or rebuilding of the cartilage.

## Brief Description of the Drawing

Figure 1 is a graph of the dose curve of IL1α on normal human cartilage.

## Detailed Description of the Invention

The invention herein encompasses the use of a prostaglandin of the following structure

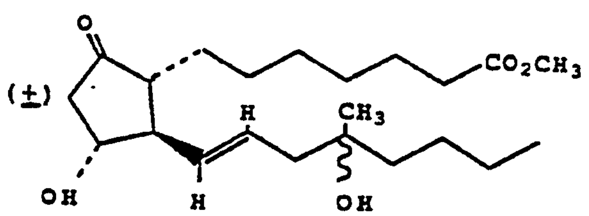

to inhibit cartilage damage and the inhibition of cartilage synthesis in patients susceptible to such damage and cartilage synthesis inhibition. The prostaglandin can be used in a method of treatment for such a patient. The method is practiced by administering to the patient susceptible to cartilage damage an effective cartilage damage inhibiting amount of the prostaglandin.

By virtue of the activity of the compounds herein in inhibiting cartilage damage, the compounds are useful in inhibiting cartilage damage which may arise as a result of a natural condition such osteoarthritis or rheumatoid arthritis or a provoked condition such as can occur by administration of NSAID therapy. A physician or veterinarian of ordinary skill can determine whether a subject patient exhibits or is susceptible to articular degeneration brought about by cartilage damage.

The compounds can be administered in such oral dosage forms as tablets, capsules, soft gels, pill, powders, granules, elixirs, or syrups.

The compounds can also be administered intravascularly, intraperitoneally, subcutaneously, intramuscularly, intraarticularly, or topically using forms known to the pharmaceutical art. Moreover, they can be administered rectally or vaginally, in such forms as suppositories or bougies. In general, the preferred form of administration is oral. For the orally administered pharmaceutical compositions, the prostaglandin will typically be administered in a mixture with suitable pharmaceutical diluents, excipients, or carriers (collectively referred to herein as "carrier" materials) suitably selected with respect to the intended form of administration. That is, oral tablets, capsules, soft gels, elixirs, syrups, drops and the like, and consistent with conventional pharmaceutical practices.

For example, for oral administration in the form of tablets or capsules, a therapeutically effective amount of one or more compounds of the present invention can be combined with any oral non-toxic pharmaceutically acceptable inert carriers such as lactose, starch, sucrose, cellulose, magnesium stearate, dicalcium phosphate,

calcium phosphate mannitol, and the like, or various combinations thereof. For oral administration in liquid forms, such soft gels, elixirs, syrups, drops and the like, a therapeutically effective amount of an active prostaglandin can be combined with any oral non-toxic pharmaceutically acceptable inert carriers such as water, saline, ethanol, polyethylene glycol, propylene glycol, corn oil, cottonseed oil, peanut oil, sesame oil, benzyl alcohol, various buffers, and the like, or various combinations thereof. Moreover, when desired or necessary suitable binders, lubricants, disintegrating agents, and prilling agents can also be incorporated in the mixture. Suitable binders include starch, gelatin, natural sugars, corn sweeteners, natural and synthetic gums such as acacia, sodium alginate, carboxymethylcellulose, polyethylene glycol, and waxes, or combinations thereof. Lubricants for use in these dosage forms include boric acid, sodium benzoate, sodium acetate, sodium chloride, and the like, or combinations thereof. Disintegrators include, without limitation, starch, methylcellulose, agar, bentonite, guar gum, and the like, or combinations thereof. Sweetening and flavoring agents and preservatives can also be included where appropriate. For intravascular, intraarticular, intraperitoneal, subcutaneous, or intramuscular administration, one or more compounds of the present invention can be combined with a suitable carrier such as water, saline, aqueous dextrose, and the like. For topical administration, therapeutically effective amounts of one or more compounds can be combined with pharmaceutically acceptable creams, oil, waxes, gels and the like.

Regardless of the route of administration selected, the prostaglandins herein are formulated in a pharmaceutically acceptable dosage form by conventional methods known to those skilled in the art.

Regardless of the route of administration selected, a non-toxic therapeutically effective quantity of one or more compounds is employed in the treatment. The dosage regimen for preventing inhibition of cartilage synthesis or inhibiting cartilage damage is selected in accordance with a variety factors, including the type, age, weight, sex, and medical condition of the patient, the severity of the cartilage damage, the route of administration, and the particular compound employed in the therapeutic regimen. A physician or veterinarian of ordinary skill can readily determine and prescribe the effective amount of the drug required to prevent or arrest the progress of the condition. In so proceeding, the physician or veterinarian can employ relatively low doses at first and subsequently increase the dose until a maximum response is obtained. Daily dosages of the compound for use in the present invention are ordinarily in the range of about 50 to about 800 milligrams/kilogram.

The compounds herein can be combined with a variety of pharmaceutically acceptable carriers and administered in a variety of dosage forms such as pills, tablets and pre-formulated liquids as well as sustained dosage forms.

A particularly preferred stable solid dosage form of the compound (+/-) methyl 11α,16-dihydroxy-16-methyl-9-oxoprost-13E-en-1-oate is a stabilized formulation as disclosed in U.S. Patent 4,301,146. The formulation disclosed in the patent is the commercially available stabilized formulation for misoprostol (the USAN name for the above named chemical compound). The commercially available misoprostol is stabilized with hydroxypropylmethylcellulose (HPMC) as set forth in the patent. For the purposes of the use of misoprostol, the commercially available misoprostol is acceptable for use in the invention herein.

The utility of the prostaglandins herein for inhibiting cartilage damage was shown in the following studies.

Example 1

A standard organ culture method was followed. Pieces of articular cartilage (4x1x1 mm) are cultured in DMEM and 5% fetal calf serum in an atmosphere of 5% $CO_2$ at 30°C. The culture medium was renewed every two days in an attempt to maintain the level of the compound undergoing evaluation. Integrity of the cartilage was determined by the retention of GAG using methylene blue dye technique, results were expressed as percentage remaining in the tissue at the end of the experiment. The synthetic activity of the chondrocytes was measured by the incorporation of $^{35}SO_4$ into GAG. In some of the evaluations incorporation of 3H-leucine into protein was measured as an index of cell viability.

The results of the experiment carried out using pig articular organ cultures are summarized in Table 1. Human recombinant IL1 alpha at a concentration of 1 ng/ml (nanograms/milliliter). At this concentration it was responsible for 68% loss in GAG content over the 8 day period whereas the control with either misoprostol vehicle or misoprostol at 200 ng/ml only lost between 4 and 7%. The cartilage was incubated in the presence of IL1 and misoprostol at 50, 100 and 200 ng/ml. At 50, 100 and 200 ng/ml there was a highly significant inhibition of the IL1 effect. At 100 ng/ml the GAG loss is reduced to 34 percent. The uptake of $^{35}SO_4$ into GAG showed a similar effect.

The results demonstrate that doses of 50 and 100 ng/ml, misoprostol is capable of significant inhibition of IL1-induced damage to articular cartilage. The inhibition is not complete but may be due to variable levels of misoprostol in the culture medium. The tissue medium was changed every two days but misoprostol exhibits a short half life which may account for low levels of misoprostol being present when compared with relatively

stable IL1 which was present throughout the experimental period. The effect of misoprostol is seen both GAG retention and on the inhibition of sulphate uptake by IL1. The experiment also shows that misoprostol has no direct affect on intact cartilage.

Table 1

**Effect of Misoprostol in IL1-treated cartilage**

| hrIL1α 1ng/ul + 0 Miso. | IL1 + Miso. 50ng/ml | IL1 + Miso. 100ng/ml | IL1 + Miso. 200ng/ml | 0IL1 + Miso. 200ng/ml | 0IL1 + Miso. Vehicle |
|---|---|---|---|---|---|
| **% Retention of GAG in tissue** | | | | | |
| 31.8 (1.8) | 46.6+ (2.8) | 65.4++ (4.5) | 45.2+ (3.2) | 95.6 (1.9) | 92.6 (2.5) |
| **Uptake of 35 $SO_4$ into GAG, cpm/mg x $10^3$** | | | | | |
| 5.9 (0.5) | 8.8+ (0.9) | 12.3++ (1.8) | 6.3 (0.8) | 29.6 (1.2) | 38.6 (2.0) |
| **Uptake of $^3H$ Leucine, cpm/mg** | | | | | |
| - | - | - | - | 70.9 (4.8) | 98.8 (4.7) |

(SEM)
\+ $p < 0.01$
++ $p < 0.001$

Example 2

The determination of the hrIL1α dose curve on human articular cartilage. This experiment was performed to investigate the action of misoprostol on the dose response of IL1 in addition to the synthesis of GAG by human cartilage. The synthesis of GAG is recognized as important in the maintenance and repair of human cartilage.

A 15 year old male patient was used to provide the articular cartilage in study A. In the subsequent study B, cartilage from a 24 year old male was used. Conventional culture conditions were used in both studies, i.e., 8 days duration with medium change at 4 days and a final pulse with 5n Ci/ml of $^{35}SO_4$. The incorporation of the isotope in GAG was measured after CPC precipitation. The initial study A had a dose range of 2.5-20 ng/ml of hrIL1α. In subsequent work the dose curve was from 0.01-20 ng/ml of hrIL1α. In study B misoprostol's affect on 2.5 ng/ml of hrILα was investigated. A total of 70 replicates were set up for study A and 90 replicates for study B. The results of the study are shown in Figure 1. Consideration of the graph demonstrates that the affect of the cytokine IL1 alpha is quite remarkable. At only 0.01 ng/ml of hrIL1α approximately 50% inhibition was observed. At 0.5 ng/ml of hrIL1α there was almost complete inhibition of GAG synthesis. As can be seen from Figure 1 the dose curves for inhibition of synthesis and GAG release, on normal human cartilage was unexpected. The inhibition of synthesis with low doses of IL1 point to the importance of the cytokine IL1 in preventing normal repair. If episodic release of low levels of the cytokine IL1 are a part of osteoarthritis pathology, the IL1 will be capable of causing, in the long term, articular damage without increased catabolic action, i.e. by preventing the normal synthesis which is essential for the maintenance of the integrity of articular cartilage in the presence of normal turnover.

Example 3

The effect of misoprostol on the action of IL1 on human articular cartilage. This experiment was undertaken to determine the action of misoprostol on IL1 action on cartilage. A total of 160 replicates were performed. The study investigated the inhibition of synthesis of GAG by human articular cartilage over the IL1 concentration range 0.01 - 1 ng/ml. Misoprostol was investigated in the range of 0.1 to 100 ng/ml.

Cartilage from a 22 year old male was used in this experiment. The experimental period was kept to 4 days with the medium being changed every 24 hours. The tissue was pulsed at 4 days with 5 μCi/ml $^{35}SO_4$ in the conventional manner and its incorporation in GAG measured after CPC precipitation.

The results of the study are shown in Table 2. It will be seen from Table 2 that 0.01 ng/ml of hrIL/1α gave approximately 50% inhibition of GAG synthesis. Complete inhibition was obtained between 0.1 and 1 ng/ml IL1. At the 50% inhibition level with IL1, misoprostol was effective. It can be seen from the Table that there is significant protection at 10 ng/ml and at 100 ng/ml. There was complete protection against the action of 0.01 ng/ml hrIL1α. Even at the lowest levels of misoprostol, misoprostol exhibited some protection starting at around the 1 ng/ml level. At higher concentrations of IL1, for example 0.1 ng/ml where very substantial inhibition of GAG synthesis is seen (greater than 85%), misoprostol was ineffective until 100 ng/ml, when there was modest protective activity.

The study shows unequivocally that misoprostol is capable of preventing the action of hrIL1α in inhibiting the synthesis of GAG in the articular cartilage from a normal human adult. The levels of IL1 used in this study are less than those seen in the fluids of patients with acute effusions (0.5 - 5 ng/ml IL1 beta[buff];1, 2 ng/ml [Nuri, et al.]), but are considered realistic for chronic disease. From the dose response curve it can be seen that even low levels of IL1 can well be expected to have a substantial affect on the integrity of human cartilage (especially in the long run.), if the release of IL1 either episodically or consistently is maintained over a period of time. The prevention of IL1's action by misoprostol emphasizes the use of misoprostol in maintaining tissue integrity.

## Table 2

**The effect of Misoprostol on IL1-induced inhibition of GAG synthesis by human articular cartilage**

$^{35}SO_4$ uptake into GAG (cpm/mg)

Misoprostol concentration ng/ml

| IL1 conc. ng/ml | 0 | 0.1 | 1.0 | 10.0 | 100.0 |
|---|---|---|---|---|---|
| 0 | 5737 (306) | - | - | - | - |
| 0.01 | 2591 (215) | 3086 (306) | 2919 (244) | 3532+ (280) | 5560++ (541) |
| 0.1 | 966 (108) | 1113 (119) | 1062 (71) | 1102 (165) | 1442+ (74) |
| 1.0 | 209 (31) | 183 (23) | 171 (14) | 219 (28) | 193 (26) |

Sig. diff. from control

\+ p = 0.01
++ p = 0.001

(SEM)

### Claims

1. The use of misoprostol in the preparation of a medicament to inhibit cartilage damage.

2. The use of a prostaglandin of the formula:

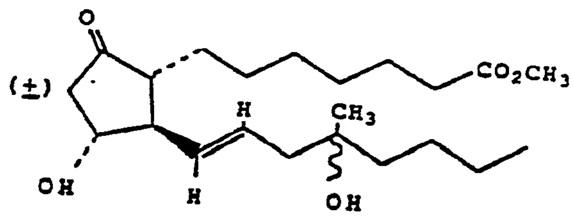

in the preparation of a medicament to inhibit cartilage damage.

3. The use of (+/-) methyl 11α, 16-dihydroxy-16-methyl-9-oxyprost-13E-en-1-oate in the preparation of a medicament to inhibit cartilage damage.

4. The use of misoprostol in the preparation of a medicament to reverse inhibition of cartilage synthesis in a patient susceptible to such damage.

5. The use of a prostaglandin of the formula:

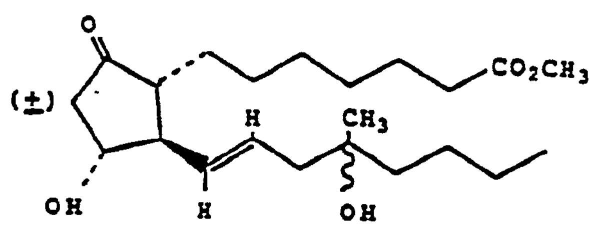

in the preparation of a medicament to reverse inhibition of cartilage synthesis in a patient susceptible to such damage.

6. The use of (+/-) methyl 11α, 16-dihydroxy-16-methyl-9-oxyprost-13E-en-1-oate in the preparation of a medicament to reverse inhibition of cartilage synthesis in a patient susceptible to such damage.

7. The use of misoprostol in the preparation of a medicament for inhibiting the damaging effect of IL1 on cartilage integrity by direct damage or inhibition of cartilage synthesis.

8. The use of a prostaglandin of the formula:

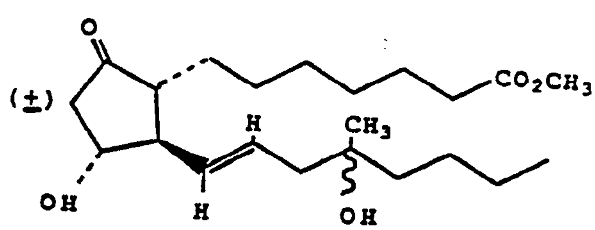

in the preparation of a medicament for inhibiting the damaging effect of IL1 on cartilage integrity by direct damage or inhibition of cartilage synthesis.

9. The use of (+/-) methyl 11α, 16-dihydroxy-16-methyl-9-oxyprost-13E-en-1-oate in the preparation of a medicament for inhibiting the damaging effect of IL1 on cartilage integrity by direct damage or inhibition of cartilage synthesis.

Figure 1

$^{35}SO_4$ Uptake Into GAG cpm/mg x $10^3$

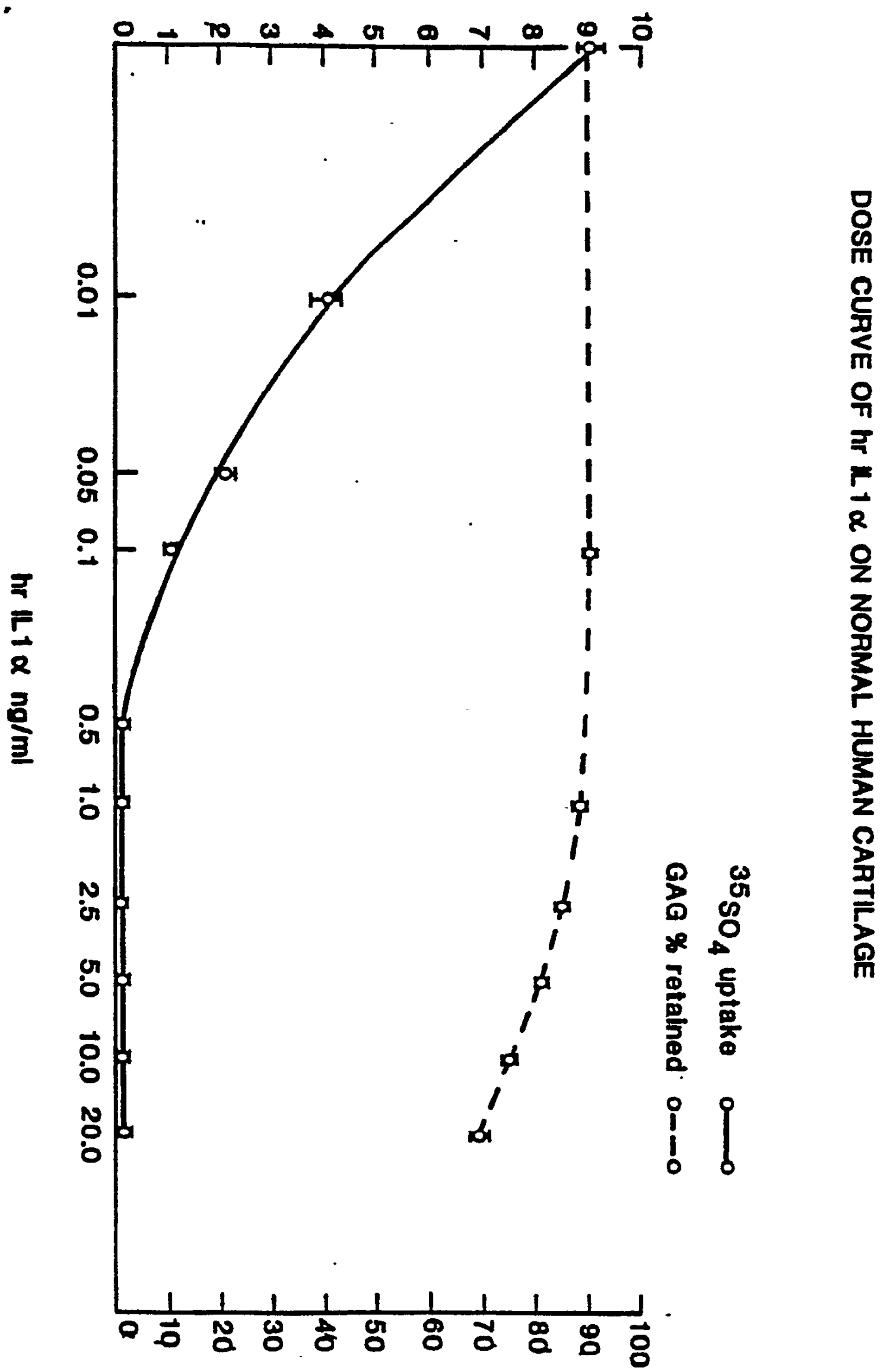


GAG Percentage Retained in Cartilage